# EUROPEAN PATENT SPECIFICATION

(11) **EP 0 395 630 B1**
(45) Date of publication and mention of the grant of the patent: **13.10.1993**
(21) Application number: 87902861.1
(22) Date of filing: 12.05.1987
(51) Int. Cl.: A61F 2/62

(54) **AN ARTIFICIAL LEG**
BEINPROTHESE
JAMBE ARTIFICIELLE

(30) Priority: 12.05.1986 SE 8602143
(43) Date of publication of application: 07.11.1990
(73) Proprietor: Lindhs Ortopediska AB, 182 34 Danderyd (SE); CENTRI GUMMIFABRIK AB, S-175 21 Järfälla (SE); RENNERFELT, Gustav Bernhard, S-181 47 Lidingö (SE)
(72) Inventor: RENNERFELT, Gustav Bernhard, S-18147 Lidingö (SE)
(74) Representative: Svanfeldt, Hans-Ake
(86) International application number: PCT/SE87/00233
(87) International publication number: WO 87/06819

(56) References cited:
- FR-A- 2 410 998
- GB-A- 2 141 345
- SE-A-83 034 157
- SE-B- 360 257
- US-A- 3 659 294

## Description

### FIELD OF THE INVENTION

The present invention relates to a prosthesis connection device including an adjusting head arranged in the proximal end of a prosthetic leg and intended for attaching the prosthetic leg to a socket mounted on the prosthetic leg for adjusting the angular as well as translational position of the prosthetic leg relative an imagined load line as specified in the preamble of Claim 1. Such a prosthesis is known from either of FR-A-2 410 998 or GB-A-2 141 345.

### DESCRIPTION OF RELATED ART

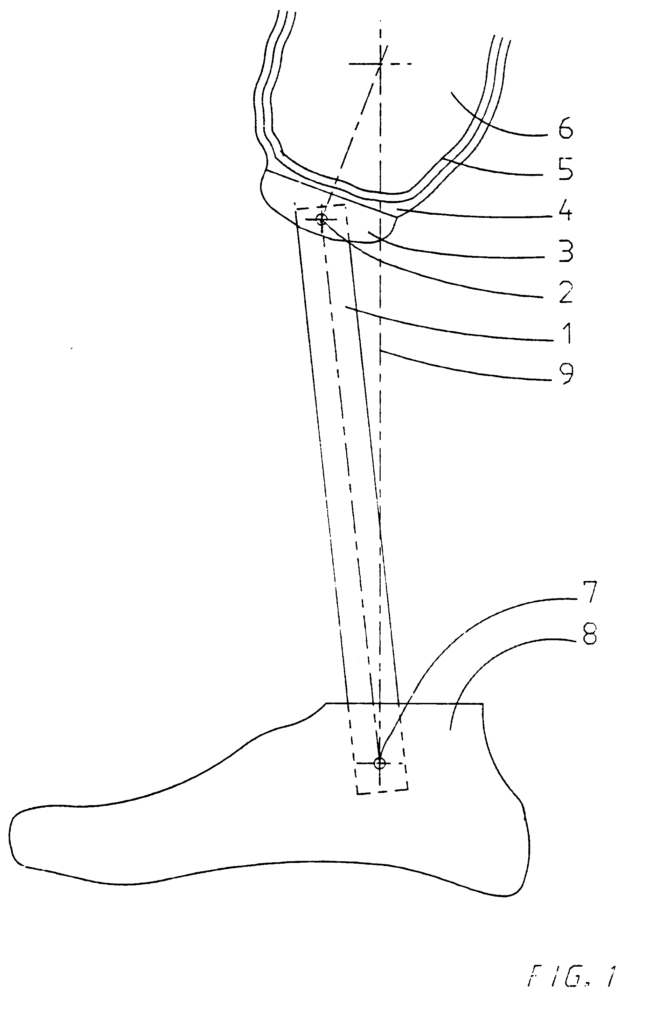

A prior art leg prosthesis is illustrated in Figure 1 and has a tube 1 which is upwardly articulately attached to a head 3 via a first joint 2, the head 3 in turn being attached to an outer socket 4, which is arranged on an inner lining 5, which is pushed over the stump 6. Downwardly, at the distal end of the tube, there is a second articulate joint 7 attached to a foot 8. The chain-dotted line 9 denotes the so-called load line, i.e. an imagined line extending between the sole of the foot (represented by the joint 7), the knee and the gravity center of the body, the body weight ideally acting along this line.

An incorrect adjustment means that the amputee loads the leg unnaturally, which results in that when the amputee walks the movement pattern of the prosthesis will be unnatural, and also that where there is a knee joint, it and the leg muscles will be unnaturally loaded. In connection with the manufacture of the socket 4, the head 3 is moulded in plastics in the best possible position relative the load line 9. In the final walking test, an adjustment must ususally be made, partly with the aid of the articulate joint 2 so that the joint 7 coincides with the load line 9, and partly with the joint 7 so that the foot is given the best attitude relative the ground plane. The structure can be likened to a tube with lockable universal joints at each end.

The disadvantage with this design principle is that the distal adjustment means, i.e. the joint 7, has a weight which gives an undesirable, distal loading moment on the stump when walking. A clear tendency in orthopedics is that the artificial foot is made as light as possible and that possible adjustment means are placed in the upper part of the prosthesis.

If the entire adjustment means is to be placed in the proximal or upper end of the prosthesis, and if it is to have the same adjustment facilities as in the structure desribed above, it must have both an angular adjustment facility such as the articulate joint 2 and a rectilinear translatory adjustment facility substantially in the horizontal plane.

There are structures where the final prosthetic leg is substituted during trials by a temporary tube having at its upper end an adjusting instrument, e.g. a Hosmer instrument, which allows both angular and translational adjustment. After walking tests with the temporary tube prosthesis and subsequent adjustment thereof, the entire temporary tube prosthesis is placed in a fixture where the temporary tube and its adjustment instrument is replaced by the final prosthetic leg, which is moulded to the outer socket 4 with the aid of a thermosetting resin.

The advantage with this design principle (being used less and less) is that the finished prosthesis will be light. However, the disadvantages are inter alia the time-consuming extra moulding of the tube, which usually requires a further fitting visit by the patient, and the relatively heavy adjusting instrument which loads the leg during the walking trial with a weight quite different from that of the final prosthesis. When the final prosthesis tube has been moulded in, no adjustment facility remains. This latter disadvantage is serious, since, particularly in amputations, the stump changes its shape due to the changes in swelling during the healing process. It is then often necessary not only to modify the inner lining 5, but is some times also desirable to make an extra adjustment of an angular and/or translatory nature.

Another known prosthesis uses the same method with an adjusting instrument and fixture as described above, but provided the final prothetic leg with an adjustment head allowing certain limited extra adjustment facilities. The disadvantages with this known prosthesis are also the required extra fitting visit by the amputee, the heavy adjusting instrument, and the work in connection with placing the prosthesis in the fixture.

When the prosthesis is finally tried out and ready, it is clothed in an individual cosmetic casting of foamed plastics or rubber material.

GB-A-2 141 345 relates to an adjustable connection device for adjoining parts of a prosthesis. The connection device comprises an upper and an lower connection member. The upper connection member allows for angular setting of the prosthesis and comprises a pin member moulded in the lower portion of a socket. The pin member is provided with a lower support surface that co-acts with an upper spherical support surface provided in the upper part of a vertically extending, generally cylindrical housing. Two pairs of set screws, angularly displaced by 90° are used to set the angular position of the prosthesis and to hold the prosthesis in its set position. The lower connection member allows for translatory movement of the prosthesis in relation to the load line and comprises a pin member which is connected to the upper portion of the prosthesis and is provided with an upper plane surface that coacts with a lower plane support surface provided in the lower portion of the cylindrical housing. Two pairs of set screws are also used to set and to hold the translatory position of the prosthesis.

This known connection device has certain drawbacks that restricts its use.

The cylindrical housing is high, restricting the use of the prothesis to short stumps where sufficient vertical space is available between the stump and the artificial foot. It cannot be used for long amputated legs having a short available vertical space between the artificial foot and the lower part of the leg. If the cylindrical housing is heavy it negatively influences walking.

In this known connection device the range of the angular setting is not sufficient from practical point of view in that it is restricted to the order of about ± 5 degrees. Therefore a special fixture has to be used before the final alignment adjustment. Should the range of angular settings be enlarged, then the size of the connection device must be enlarged, thus increasing the size and the weight of the connection device which is undesired from cosmetic and walking pattern point of view respectively.

The maximum amount of translatory movement allowed for by this known device is relatively small especially in view of the fact that 2-3 mm are required for a compensating translatory movement, required in consequence of the combined rotation and displacement of the pin of the upper connection member upon angular setting thereof, leaving only a few mm for translatory setting of a correct loading line, this small amount being far to small if the person for example is knock-kneed. In this case the upper portion of the prosthesis must be moved a large translatory distance in order to bring the lower portion of the prosthesis in a position vertically under the knee thereby maintaining the proper load line. Also translatory movements of the prosthetic leg along a direction which forms an angle, e.g. 5°, 10° or 15°, with the walking direction are difficult, if not impossible, to perform with this known device.

Moreover the translatory movement is taking place in the lower connection member which is situated at a distance beneath the angular setting means implying that a large compensating translatory movement of the prosthetic leg must be made even for small changes of the angle of inclination of the prosthetic leg.
The fact that the pin is moulded into the socket in this known device poses problems. Often it is desired that the two screws of one of the two pairs of set screws for setting the angular position shall lay along a line that coincides with the walking direction while the set screws of the second pair of set screws for setting the angular position shall be provided along a line perpendicular to the walking direction. This often desired rotational position of the set screws facilitates the angular adjustment of the prosthetic leg. It is therefore required that the rotational position of the pin relative to the socket is carefully set before moulding the pin to the socket, since no later on adjustments of the rotational position of the pin can be made. If the correct rotational position of the pin is missed the moulding procedure must be repeated.

The fact that the pin is moulded into the socket also means that a vital, and expensive, part of the connection device has to be replaced each time the socket is replaced. As pointed out above socket replacements are frequent and take place whenever required due to changes of the stump form.

Also the angular setting of the prosthetic leg is troublesome with this known device. Once one set screw of the first pair of angular set screws has been unscrewed and the opposite set screw of the first set screw pair is tightened in order to rock the pin, movement of the pin is hampered due to friction caused by the remaining two set screws of the second pair of angular set screws, said remaining two set screws still pressing against the opposite sides of the pin. It will therefore be necessary to unscrew at least one of said remaining two set screws in order to create a clearance allowing the pin to rock between the two remaining set screws.

Accordingly at least three set screws must be manipulated which is time consuming. Due to said clearance the prosthetic leg is free to rock and twist during the angular setting. Such uncontrolled rocking and twisting movements may have a negative impact during setting of the final angular position of the prosthetic leg, especially if the angular setting is made while the person is wearing the prosthesis.

French patent 2 410 998 relates to a prosthetic leg with means for adjusting the angular as well as translatory position of the prosthesis. The angular setting means comprise two obliquely cut blocks rotably mounted for mutual movement around a spherical nut and screw assembly. The nut and screw assembly in turn is slidably mounted within an opening provided in a mounting plate thereby allowing for translatory movement of the prosthesis within the confinment of said opening. The mounting plate is connected with the socket by conventional connection means such as laths or double ferrules which may be riveted, counterbored or welded. This known device has several drawbacks.

Its angular setting means is of the kind that setting the angular position of the prosthesis in one plane, for example the vertical plane directed in the walking direction, will change the angle previously set in another vertical plane perpendicular to said first vertical plane. In order to restore the angle set in said second vertical plane second angular setting means are provided at the foot of the prosthesis. This increases the weight of the prosthetic leg and negatively influences the walking of the patient.

The height of each angular setting means of said French patent is large and this reduces the utility of said means to a long prosthesis only.

In said French patent the means for allowing translatory motion comprises a washer, covering the opening and the surrounding upper surface of the mounting plate, the angular setting means, covering the opening and the surrounding lower surface of the mounting plate, and the nut and screw assembly holding the washer and the angular setting means together pressed against the mounting plate. The nut of said nut and screw assembly is situated in the closed space formed between the mounting plate, the connection means for connecting the mounting plate to the lower surface of the socket. Accordingly, there is no access to the nut once the mounting plate has been fastened to the sleeve. It will therefore not be possible to adjust the translational position of the prosthesis once it has been mounted on the socket. No follow up adjustments can be made with this known device while the prosthesis is attached to the socket. Follow up adjustments of the translatory position are possible but require dismounting of the mounting plate from the socket and is accordingly troublesome and time consuming.

US-A-3 659 294 describes a prosthetic leg of the general kind referred to above in connection with Figure 1. The prosthetic leg has no means for providing translatory movement adjustments; the prosthetic leg is only pivotally adjustable.

An object of the present invention is to provide a prosthetic leg connecting member that reduces the drawbacks of the prior art devices and is light, low and allows for large angular as well as translatory settings of the position of the prosthetic leg at one point close to the stump even after prosthetic manufacture is finished, that is whenever needed .

Another object of the invention is to provide a prosthetic leg connection member the rotational position of which in relation to the socket may be set in any position. Moreover, follow up adjustments of said rotational position should be possible to make while the prosthetic leg and its connection member are attached to the stump and even when the amputee is standing.

Sliding movement of the prosthetic leg in any horizontal direction should be easy to perform.

In accordance with another aspect of the invention the prosthetic leg connection member has angular setting means comprising, in a manner known per se, two pairs of set screws angularly displaced by 90° and permitting angular setting of a prosthetic the leg in two orthogonal vertical planes such that the setting of an angle of inclination of the prosthetic leg in one vertical plane does not affect the setting of another angle of inclination of the prosthetic leg in the vertical plane perpendicular to the first mentioned vertical plane.

A further object of the invention is to arrange the angular setting means so that only the two set screws of one pair need to be operated upon setting of the angle of inclination of the prosthetic leg connection member in the vertical plane in which said pair of set screws are provided. While the two set screws of one pair are operated upon the set screws of the remaining pair will slide in cylindrical grooves stationary relative to a support plate of the prosthetic leg connection member thereby stabilizing the prosthetic leg connection member and providing for a controlled angular motion thereof in said vertical plane.

### BRIEF DESCRIPTION OF THE DRAWINGS

A preferred embodiment of the invention will now be described in detail below and with reference to the accompanying drawings which are given by may of illustration only and thus are not limitative of the present invention and wherein,
Figure 1 is a schematic side view of a known prosthetic leg,
Figure 2 is a side view of the prosthetic leg connection member, in accordance with the invention,
Figure 3 illustrates the prosthetic leg connection member in a sectional view,
Figures 4, 5 and 6 illustrate a sleeve included in the prosthetic leg connection member in axial section, plan from above and side view.

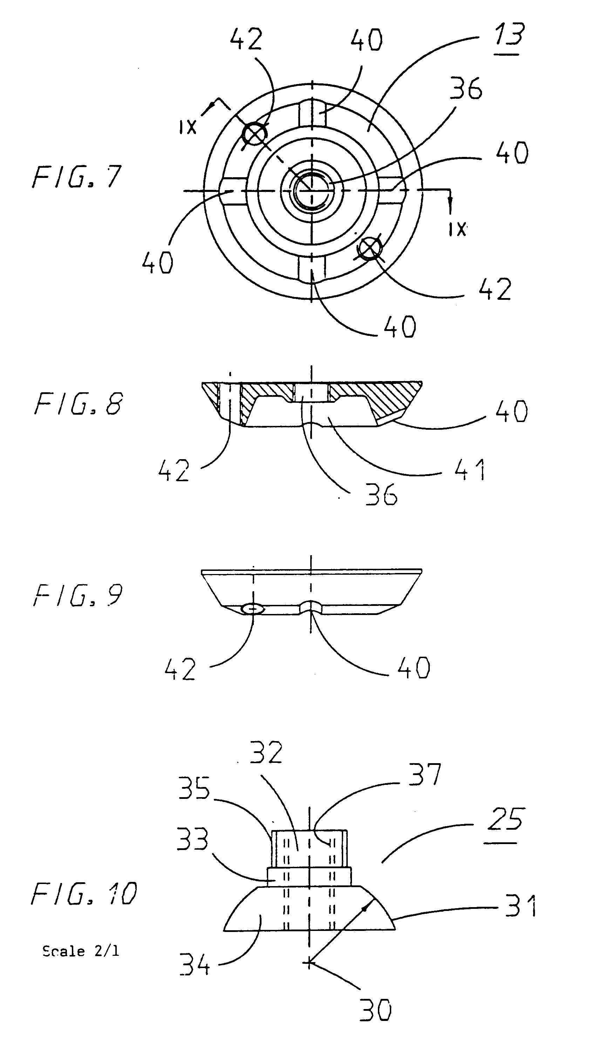

Figures 7, 8 and 9 illustrate a carrying plate included in the prosthetic leg connection member in plan, section along the line IX-IX in Figure 7 and side view,
Figure 10 is a side view of a hemispherical nut included in the prosthetic leg connection member in accordance with the invention.

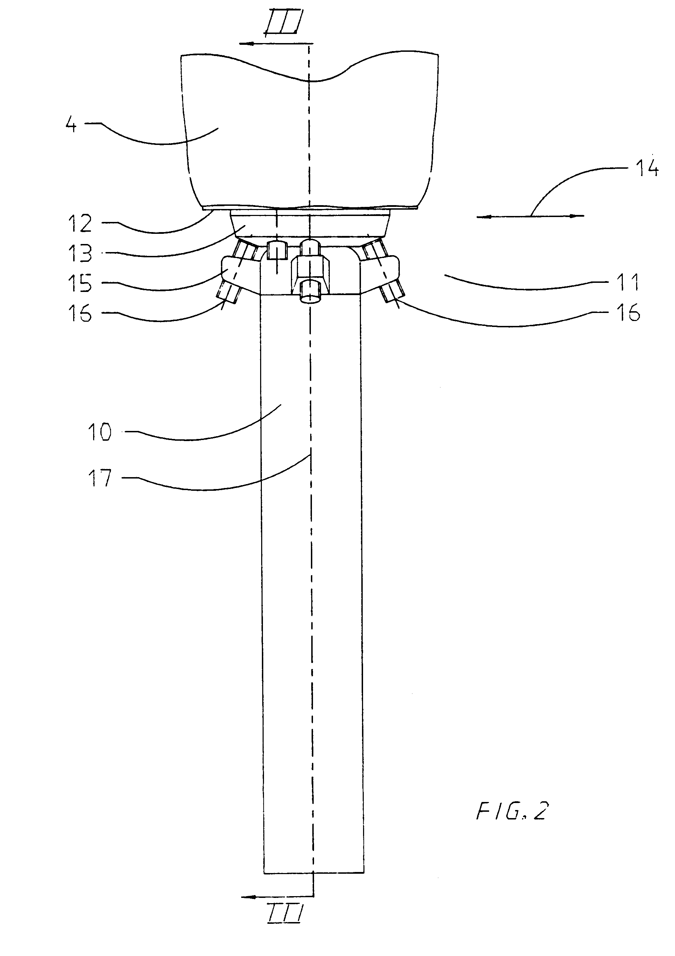

Figure 2 is a side view of a prosthetic leg connection member 11 in accordance with the invention. It connects a prosthetic leg, in the form of a tube 10, with an outer socket 4 for the stump, not shown. The prosthetic leg connection member 11 includes an upper flat ring 12 and a lower flat carrying plate 13, the ring and plate being mutually relatively slidable, not only in the direction denoted by the arrows 14 but in all other horizontal directions in the interface between them. The head 11 also includes a sleeve 15, which is fastened to the proximal end of the tube 10 and has a plurality of adjusting screws 16, the function of which will be described in more detail below. The longitudinal axis of the tube 10 is denoted by the numeral 17.

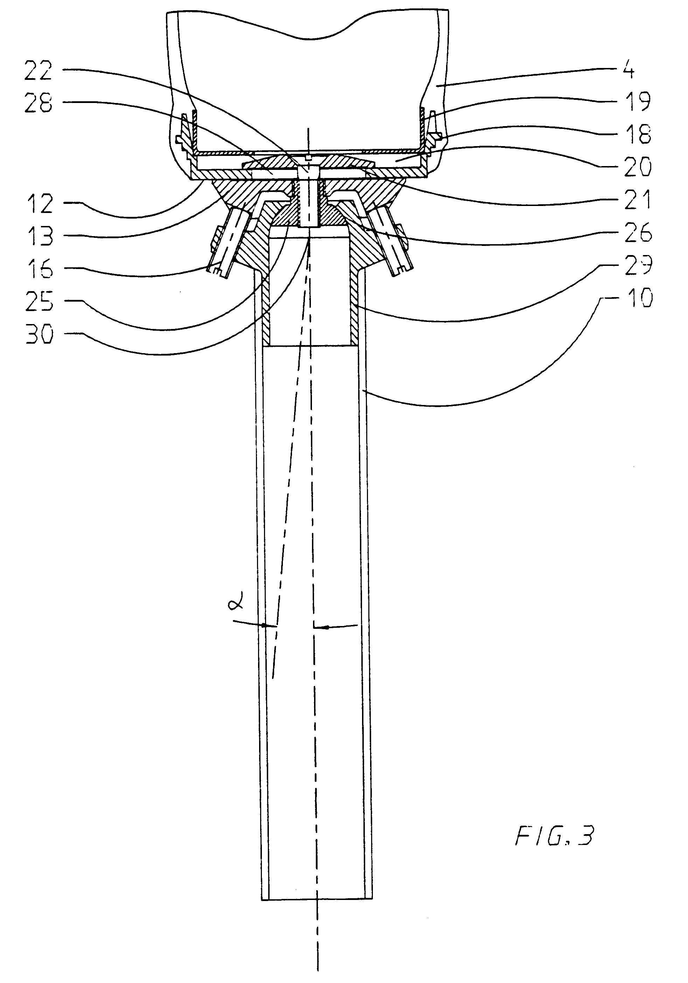

Figure 3 is a sectional view along the line III-III in Figure 2, of the prosthetic leg connection member in accordance with the invention. As will be seen from Figure 3, the ring 12 includes a flange 18 integral with the ring and provided with a serrated exterior contour. The socket 4 is open-ended and the flange 18 is moulded into the wall of the socket 4. On the inside of the flange there is a shoulder, on which rests a cover 19, for protection against the penetration of moulding composition into the space 20 formed under the cover. After moulding the ring 12 into the wall at the open end of the socket this cover may selectively be discarded or may be retained. In the latter case the center portion of the cover is made of a material which can be pierced by for example a screw driver. On the upper side of the ring 12 there is a holding-down plate 21 with a central hole. A screw 22, with its head resting on this plate, is threaded into a hemispherical nut 25, which has its hemispherical surface 26 engaging slindingly against the complemental surface in the upper end portion 27 of the sleeve 15. On tighthening the screw 22 this arrangement urges the plate 21 towards the lower carrying plate 13 such that these two bear against the ring 12. The holding-down plate 21 and carrying plate 13 are preferably circular, while the ring 12 has a central circular opening 28 allowing translatory movement of the tube 10. The dimensions of the ring 12, the circular opening 28 and the diameter of the holding-down plate 21 are such that the prosthetic leg connection member 11, and thus also the tube 10, can slide ± 12.5 mm in all directions in the common plane between ring and carrying plate from the position illustrated in Figure 3.

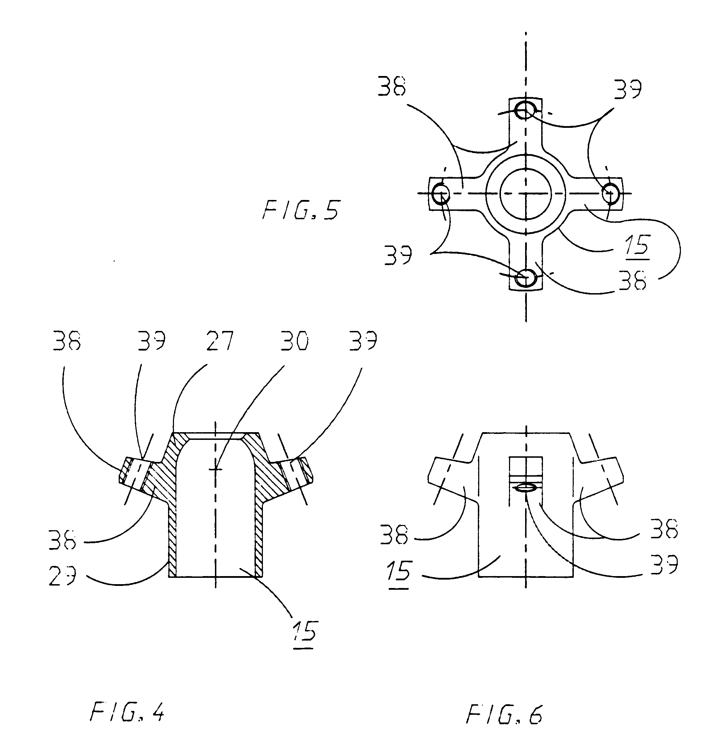

The sleeve 15, illustrated in different views in Figures 4-6, has a short throat 29, which is slidingly fit into the tube 10 and it locked by suitable, unillustrated means, e.g. pop rivets, to the tube 10. The internal wall of the upper end portion 27 of the sleeve 15 is rounded off, and in section has the form of a spherical sector with its centre indicated by the numberal 30. The spherical portion of the hemispherical nut 25 has, as illustrated in Figure 10, its surface denoted by 31, and is complemental to the spherical surface 26 on the upper end portion 27, thus signifying that the surfaces 26 and 31 have the same radii and centre 30, which in its turn is situated in the throat 29 and coincident with the longitudinal axis 17 of the tube 10, as illustrated in Figure 3. As shown in Figure 10 the hemispherical nut 25 comprises a stub shaft 32 with a shoulder 33 and a spherical segment 34. The stub shaft 32 has an external thread 35 which meshes with a threaded central opening 36 in the carrying plate 13 (see Figure 8). The nut is also provided with a through internal thread 37 for the screw 22.

The angular adjustment of the longitudinal axis 17 of the tube 10 is perhaps easiest to understand if it is first imagined that the adjustment screws 16 are completely removed (which is a condition not normally present). The tube 10, retained by the sleeve 15 may then be set at different angular positions with the internal surface of the upper part 27 of the sleeve 15 sliding on the stationary spherical segment surface 31. The angular adjustment movements of the tube 10 can thus take place by rotation round an imaging rotational axis, which always passes through the centre 30. It will thus be clear that the tube can be pivoted universally around the centre 30. In the illustrated embodiment of the invention, the angular setting of the tube can be adjusted ±12° in all directions from the attitude illustrated in Figure 3.

A description of how the angular position of the tube 10 is adjusted and locked with the adjusting screws 16 in place will now be given. A plurality of arms 38 extend radially outwards from the wall of the sleeve 15 in the region of the bottom of the upper end portion 27. The arms form an angle of 68° with the longitudinal axis of the sleeve and slope upwards towards the level of the upper end portion 27. The arms, which are integral with the sleeve, are each provided with a threaded through hole 39 for receiving the adjusting screw 16.

These adjusting screws 16 are rounded at their forward ends and engages against grooves 40 made in the lower surface of the carrying plate 13. In its central portion the plate has a recess 41 for accommodating the end portion 27 (Figure 4) of the sleeve 15. In the illustrated embodiment, the number of arms 38 and grooves 40 is preferably four and they are arranged with uniform distribution, i.e. 90°, about the centre of the thread opening 36. In cross section, the grooves 40 have the form of circular or elliptical sectors, and the holes 39 are inclined relative the longitudinal axis of the sleeve, such that screwing in the left hand screw 16 in Figure 3, accompanied by a corresponding loosening of the right hand screw, results in that the other two screws not illustrated in Figure 3, since they are in a plane at right angles to the paper, slide on the surface of their respective grooves 40 with the centre of this pivoting movement situated at the centre 30. The movement is such that the forward ends of the two unillustrated screws glide on and follow the contour of the grooves 40. The arms in which said two not illustrated screws are provided will thus keep the tube 10 stabilized during its angular motion. The angular adjustment of the tube is thus facilitated, since these two unillustrated screws do not need to be loosened when the screws 16 in Figure 3 are rotated contra each other. It will be understood that apart from locking the set angular position, the screws will also allow fine adjustment of the angular position of the tube. An important finesse with the invention is that the angular position of the tube can be adjusted even when the screw 22 has been finally tighthened. It is thus possible, after the prosthesis has been in use for some time, to adjust its angular position without needing to remove it from the amputee for the purpose of allowing access to the screw 22. That such later adjustment is possible will be understood from the fact that the hemispherical nut 25 is screwed down on the carrying plate 13 to form a single unit with it. Tightening the screw 22 fixes the translatory position of the carrying plate 13, since the ring 12 is firmly clamped between the holding-down plate 21 and the carrying plate 13. Tightening the screw 22 thus does not affect the possibility of angularly adjusting the tube with the adjusting screw 16.

By using four adjusting screws 16, four arms 38 and four grooves 40, all in uniform angular spacing, i.e. 90°, there is achieved the additional advantages that the angle, e.g. in Figure 3 is set for the tube in one plane by complementally screwing the screws illustrated in Figure 3, an adjustment of the tube angle in a plane at right angles to that of the paper can then be undertaken by complemental screwing of the unillustrated screws 16, without the previously set angle being altered. Should only three arms, screws and grooves at 120° to each other be used, a previously set angular position for one screw would be changed by an adjustment of the tube angle in another plane and the setting would need to be readjusted afterwards.

The carrying plate 13 also has two through threaded holes 42 for receiving two locking screws, unillustrated in Figures 2 and 3, with the aid of which the adjustment of the support plate 13 to the ring 12 can be temporarily fixed during trying-out the prosthesis until the correct translatory position has been obtained, these screws then being tightened finally. Any possible later angular adjustment by the screws 16 is not obstructed by this final fixation of the translatory position.

The basic setting of a prosthetic leg with the aid of the prosthetic connection member in accordance with the invention takes place in the following manner
1) The socket of the prosthetic leg is attached to the stump, the tube is adjusted to a vetical position.
2) The tube is translated so that it coincides with the load line.
3) The unillusrated locking screw in the threaded holes 42 of the carrying plate 13 are tightened.
4) The amputee performs a walking test.
5) The angular setting of the tube is adjusted with the adjusting screw 16, so that the tube does not move from side to side in the vertical plane during walking. Items 4) and 5) may be repeated.
6) The head locking screw 22 is tightened.
7) The whole adjusting head may optionally be moulded-in while the patient waits. The adjusting screws 16 can also be covered with a thin and easily removable plastic layer.

## Claims

1. A leg prosthesis connection device including a tube (10), an adjusting head (11) arranged at the proximal end of the tube and intended for attaching the prosthesis to a first sleeve (4) arranged on the leg stump (6), first means (16, 38, 40) for adjusting the angular position of the tube relative an imagined load line (9), second means (12, 13, 21) for allowing a translatory movement of the tube, and fixing means (22, 25) for fixing the tube in a desired translatory position relative the first sleeve (4), said first and second means both being situated on the adjusting head at the proximal end of the tube and both allowing basic adjustment of the tube with the prosthesis in place on the patient, **characterized in that** said first means comprise a second sleeve (15) fixed to the proximal end of the tube (10), said second sleeve including a throat (29) provided with a plurality of radially projecting arms (38) arranged on the throat to form an angle with the longitudinal axis of the second sleeve, and a threaded through hole (39) in the end portion of each arm, an adjusting screw (16) with a rounded end received in each hole (39), said rounded end engaging against abutments of a lower surface of a support plate (13) which is disposed below said first sleeve (4) and forms a part of said second means (12, 13, 21).

2. A leg prosthesis connection device in accordance with claim 1, **characterized in that** said second sleeve (15) has a proximal end portion (27), the interior of which has the shape of a circle sector in cross section so as to form a surface (26) shaped as a spherical segment.

3. A leg prosthesis connection device in accordance with claim 2, **characterized in that** said support plate (13) has a central, through hole (36) for the fixing means and wherein said abutment take the form of radially directed grooves (40) enabling the interface member to take up torque acting on its symmetrical axis.

4. A leg prosthesis connection device as claimed in claim 3, **characterized in that** said grooves (40) have a circular or elliptical sector as their cross sectional shape, the center of said sector being substantially in a horizontal plane crossing the center (30) of the spherical surface (26) of the second sleeve (15).

5. A leg prosthesis connection device as claimed in claim 4, **characterized in that** said fixing means comprises a hemispherical nut (25) and a fixing screw (22), said hemispherical nut including a stub shaft (32) with an external thread (35) and a spherical segment (34) intended for engagement against the internal proximal portion of the second sleeve (15) with the stub shaft (32) projecting through said portion, said threaded stub shaft being intended for screwing into the through hole (36) of the support plate (13), said spherical nut also having an internal thread (37) intended for meshing with said fixing screw (22).

6. A leg prosthesis connection device in accordance with claim 4, **characterized in that** the number of adjusting screws (16), arms (38) on the second sleeve and grooves (40) on the support plate (13) is four and wherein said items are arranged in mutual equally spaced angular relationship, i.e. 90° from each other.

7. A leg prosthesis connection device in accordance with claim 6, **characterized in that** said second means (12, 13, 21) include said support plate (13) with a holding-down plate (21) arranged above it, and a ring (12) which is preferably circular and arranged between said support plate (13) and the holding-down plate (12), the internal diameter of the ring (12) being less than the extension of the holwing-down plate (21), said fixing means (22) being adapted for locking the ring between the holding-down plate (21) and support plate (13) and for fixing the structure formed by the holding-down plate (21), support plate (13) and ring (12) on the second sleeve (15), the ring having a projecting flange (18) directed obliquely upwardly for fixing in the lower portion of the first sleeve (4).

8. A leg prosthesis connection device in accordance with claim 7, **characterized in that** said fixing means furhter included locking screws meshing in threads (42) provided in the support plate, said locking screws being adapted to engage the ring.

## Patentansprüche

1. Vorrichtung zur Befestigung einer Beinprothese, mit einem Rohrabschnitt (10), einem Justierkopf (11), der am proximalen Ende des Rohrabschnitts angeordnet und zur Befestigung der Prothese an einer auf dem Beinstumpf (6) angeordneten ersten Muffe (4) vorgesehen ist, ersten Mitteln (16, 38, 40) für die Einstellung der Winkellage des Rohrabschnitts relativ zu einer imaginären Lastlinie (9), zweiten Mitteln (12, 13, 21), die eine translatorische Verschiebung des Rohabschnitts ermöglichen, und mit Feststellmitteln (22, 25) zur Fixierung des Rohrabschnitts in einer gewünschten translatorischen Lage relativ zu der ersten Muffe (4), wobei sowohl die ersten als auch die zweiten Mittel an dem Justierkopf am proximalen Ende des Rohrabschnitts vorgesehen sind und eine Grundeinstellung des Rohrabschnitts bei einer an den Patienten angelegten Prothese erlauben, **dadurch gekennzeichnet**, daß die ersten Mittel eine am proximalen Ende des Rohrabschnitts (10) befestigte zweite Muffe (15) aufweisen, wobei die zweite Muffe einen mit einer Mehrzahl von sich radial erstreckenden Armen (38) versehenen Hals (29) aufweist, die so auf dem Hals angeordnet sind, daß sie mit der Longitudinalachse der zweiten Muffe einen Winkel ausbilden, und daß im Endbereich jedes Arms ein durchgehendes Loch (39) mit Gewinde und in jedem Loch eine Justierschraube (16) mit einem abgerundeten Ende vorgesehen ist, wobei die abgerundeten Enden in Widerlagern an einer unteren Oberfläche einer Stützplatte (13) anliegen, die unterhalb der ersten Muffe (4) angeordnet ist und ein Teil der zweiten Mittel (12, 13, 21) ausbildet.

2. Vorrichtung zur Befestigung einer Beinprothese nach Anspruch 1, **dadurch gekennzeichnet**, daß die zweite Muffe (15) einen proximalen Endbereich (27) aufweist, dessen Inneres im Querschnitt die Form eines Kreissegments aufweist, so daß es eine Oberfläche (26) mit der Form eines Kugelsegments ausbildet.

3. Vorrichtung zur Befestigung einer Beinprothese nach Anspruch 2, **dadurch gekennzeichnet,** daß die Stützplatte (13) ein zentrales, durchgehendes Loch (36) für die Feststellmittel aufweist und daß die Widerlager die Form radial ausgerichteter Nuten (40) aufweisen, die es dem Zwischenglied ermöglichen, auf seine Symmetrieachse einwirkende Drehmomente aufzunehmen.

4. Vorrichtung zur Befestigung einer Beinprothese nach Anspruch 3, **dadurch gekennzeichnet**, daß die Nuten einen kreisförmigen oder elliptischen Abschnitt als ihre Querschnittsform aufweisen, wobei das Zentrum des Abschnitts im wesentlichen in einer horizontalen, das Zentrum (30) der kugelförmigen Oberfläche (26) der zweiten Muffe (15) schneidenden Ebene angeordnet ist.

5. Vorrichtung zur Befestigung einer Beinprothese nach Anspruch 4, **dadurch gekennzeichnet,** daß die Feststellmittel eine halbkugelförmige Mutter (25) und eine Feststellschraube (22) aufweisen, wobei die halbkugelförmige Mutter einen Schaftstummel (32) mit einem Außengewinde (35) und ein Kugelsegment (34), das zur Anlage an dem inneren Proximalbereich der zweiten Muffe (15) vorgesehen ist, aufweist, während sich der Schaftstummel (32) durch diesen Proximalbereich hindurch erstreckt, wobei der mit dem Gewinde versehene Schaftstummel zum Einschrauben in das durchgehende Loch (36) der Stützplatte (13) vorgesehen ist und wobei die kugelförmige Mutter weiterhin ein Innengewinde (37) aufweist, das zum Ineinandereingreifen mit der Feststellschraube (22) vorgesehen ist.

6. Vorrichtung zur Befestigung einer Beinprothese nach Anspruch 4, **dadurch gekennzeichnet,** daß die Anzahl der Justierschrauben (16) und der Arme (38) an der zweiten Muffe sowie der Nuten (40) in der Stützplatte (13) vier ist, wobei diese Bauteile in einer Winkelbeziehung mit gleichen Abständen untereinander, d. h. jeweils 90° voneinander entfernt, angeordnet sind.

7. Vorrichtung zur Befestigung einer Beinprothese nach Anspruch 6, **dadurch gekennzeichnet,** daß die zweiten Mittel (12, 13, 21) die Stützplatte (13) mit einer darüber angeordneten Rückhalteplatte (21) und einen Ring (12), der vorzugsweise rund ist und der zwischen der Stützplatte (13) und der Rückhalteplatte (21) angeordnet ist, einschließen, wobei der innere Durchmesser des Rings (12) kleiner als die Ausdehnung der Rückhalteplatte (21) ist, wobei die Feststellmittel (22, 25) zum Blockieren des Rings zwischen der Rückhalteplatte (21) und der Stützplatte (13) und zum Befestigen der von der Rückhalteplatte (21), der Stützplatte (13) und dem Ring (12) ausgebildeten Anordnung an der zweite Muffe (15) vorgesehen sind und wobei der Ring einen überstehenden, schräg nach oben gerichteten Bund (18) zur Befestigung im unteren Bereich der ersten Muffe (4) aufweist.

8. Vorrichtung zur Befestigung einer Beinprothese nach Anspruch 7, **dadurch gekennzeichnet,** daß die Feststellmittel weiterhin Sperrschrauben umfassen, die in in der Stützplatte vorgesehene Gewinde (42) eingreifen, wobei die Sperrschrauben zum Einwirken auf den Ring vorgesehen sind.

## Revendications

1. Dispositif de connexion d'une prothèse de la jambe comprenant un tube (10), une tête de réglage (11) prévue à l'extrémité proximale du tube et destinée à attacher la prothèse à un premier manchon (4) placé sur le moignon de jambe (6), des premiers moyens (16,38,40) pour règler la position angulaire du tube par rapport à une ligne de charge théorique (9), des deuxièmes moyens (12,13,21) pour permettre un mouvement de translation du tube, et des moyens de fixation (22,25) pour fixer le tube dans une position de translation désirée par rapport au premier manchon (4), lesdits premiers et deuxièmes moyens étant tous situés sur la tête de réglage, à l'extrémité proximale du tube, et permettant un réglage de base du tube lorsque la prothèse est en place sur le patient, caractérisé en ce que lesdits premiers moyens comprennent un deuxième manchon (15) fixé à l'extrémité proximale du tube (10), ce deuxième manchon comportant un col (29) pourvu d'une pluralité de bras radialement en saillie (38), disposés sur le col de manière à former un angle avec l'axe longitudinal du deuxième manchon, et un trou traversant taraudé (39) ménagé dans la partie d'extrémité de chaque bras, une vis de réglage (16) à bout arrondi vissée dans chaque trou (39), ladite extrémité arrondie venant en contact contre des butées prévues sur une surface inférieure d'une plaque d'appui (13) qui est disposée sous ledit premier manchon (4) et qui fait partie desdits deuxièmes moyens (12,13,21).

2. Dispositif de connexion d'une prothèse de la jambe suivant la revendication 1, caractérisé en ce que ledit deuxième manchon (15) comporte une partie d'extrémité proximale (27) dont l'intérieur a une section transversale en forme de secteur de cercle de manière à engendrer une surface (26) en forme de segment de sphère.

3. Dispositif de connexion d'une prothèse de la jambe suivant la revendication 2, caractérisé en ce que ladite plaque support (13) comporte un trou traversant central (36) pour les moyens de fixation, et en ce que les dites butées sont sous la forme de gorges radiales (40) permettant à l'élément d'interface d'absorber un couple agissant sur son axe de symétrie.

4. Dispositif de connexion d'une prothèse de la jambe suivant la revendication 3, caractérisé en ce que lesdites gorges (40) ont une section transversale en forme de secteur circulaire ou elliptique, le centre du dit secteur étant situé sensiblement dans un plan horizontal passant par le centre (30) de la surface sphérique (26) du deuxième manchon (15).

5. Dispositif de connexion d'une prothèse de la jambe suivant la revendication 4, caractérisé en ce que lesdits moyens de fixation comprennent un écrou hémisphérique (25) et une vis de fixation (22), ledit écrou hémisphérique comportant une courte tige (32) filetée extérieurement (35) et un segment sphérique (34) prévu pour venir en contact contre la partie proximale intérieure du deuxième manchon (15), la tige (32) faisant saillie à travers ladite partie, ladite tige filetée pouvant se visser dans le trou traversant (36) de la plaque support (13), et ledit écrou sphérique comportant également un filetage intérieur (37) prévu pour un accouplement avec ladite vis de fixation (22).

6. Dispositif de connexion d'une prothèse de la jambe suivant la revendication 4, caractérisé en ce que le nombre des vis de réglage (16), des bras (38) sur le deuxième manchon et des gorges (40) de la plaque support (13) est de quatre, et en ce que ces éléments sont agencés de façon angulairement équidistante les uns par rapport aux autres, c'est-à-dire à 90° les uns des autres.

7. Dispositif de connexion d'une prothèse de la jambe suivant la revendication 6, caractérisé en ce que lesdits deuxièmes moyens (12,13,21) comprennent ladite plaque support (13) et une contreplaque (21) placée audessus de la plaque support, et un anneau (12) qui est de préférence circulaire et placé entre ladite plaque support (13) et la contreplaque (12), le diamètre intérieur de l'anneau (12) étant plus petit que la dimension de la contreplaque (21), lesdits moyens de fixation (22) étant prévus pour bloquer l'anneau entre la contreplaque (21) et la plaque support (13) et pour fixer la structure, constituée par la contreplaque (21), la plaque support (13) et l'anneau (12), sur le deuxième manchon (15), l'anneau comportant une collerette en saillie (18) s'étendant obliquement vers le haut pour une fixation dans la partie inférieure du premier manchon (4).

8. Dispositif de connexion d'une prothèse de la jambe suivant la revendication 7, caractérisé en ce que lesdits moyens de fixation comprennent en outre des vis de blocage qui se vissent dans des trous taraudés (42) prévus dans la plaque support, lesdites vis de blocage pouvant venir en prise avec l'anneau.
